**Europäisches Patentamt**

**European Patent Office**

**Office européen des brevets**

(11) Publication number: **0 068 032**
**B1**

# (12) EUROPEAN PATENT SPECIFICATION

(45) Date of publication of patent specification: **24.05.89**

(21) Application number: **82900225.2**

(22) Date of filing: **04.01.82**

(86) International application number:
**PCT/JP82/00001**

(87) International publication number:
**WO 82/02333 22.07.82 Gazette 82/18**

(51) Int. Cl.⁴: **A 61 B 5/04**

(54) **ELECTROCARDIOGRAPH.**

| | |
|---|---|
| (30) Priority: **31.12.80 JP 187524/80** | (73) Proprietor: **TOYOSU, Yasuhiro**<br>**15-5, Minamikomatsushimacho Komatsushima-shi**<br>**Tokushima 773 (JP)** |
| (43) Date of publication of application:<br>**05.01.83 Bulletin 83/01** | (73) Proprietor: **AKAMATSU, Norio**<br>**1-8, Higashiyoshinocho 2-chome Tokushima-shi**<br>**Tokushima 770 (JP)** |
| (45) Publication of the grant of the patent:<br>**24.05.89 Bulletin 89/21** | (73) Proprietor: **Kawabe, Jiroh**<br>**91 Hamazaki Mugiura Mugi-cho Kaifu-gun**<br>**Tokushima 775 (JP)** |
| (84) Designated Contracting States:<br>**AT BE CH DE FR GB LI LU NL SE** | (72) Inventor: **Toyosu, Yasuhiro**<br>**15-5, Minamikomatsushimacho Komatsushima-shi**<br>**Tokushima 773 (JP)**<br>Inventor: **Akamatsu, Norio**<br>**1-8, Higashiyoshinocho 2-chome Tokushima-shi**<br>**Tokushima 770 (JP)** |
| (56) References cited:<br>**DE-A-3 004 126**<br>**DE-A-3 025 955**<br>**FR-A-2 088 780**<br>**FR-A-2 308 343**<br>**FR-A-2 431 280**<br>**JP-Y-51 001 659**<br>**JP-Y-53 034 230**<br>**US-A-3 052 232**<br>**US-A-3 896 790** | (74) Representative: **Freed, Arthur Woolf et al**<br>**Reginald W. Barker & Co. 13 Charterhouse Square**<br>**London EC1M 6BA (GB)** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

**IEEE - 1980 IECI PROCEEDINGS "APPLICATIONS OF MINI AND MICROCOMPUTERS", 17th-20th March 1980, Philadelphia, Pennsylvania, US, pages 361-366, IEEE, New York, US; N. AKAMATSU et al.: "A computerized technique for body surface isopotential and topological maps"**

## Description

The present invention relates to an electrocardiograph in which potentials of a number of points of the human body surface near the heart are measured, and through the information processing of the measured potentials, a body surface potential map at a sampled time is made so that the electrical activity of the heart can be accurately detected.

According to a most generally used electrocardiograph, the potential change of six points of the chest in lapse of time is measured and showed in a graph with time as abscissa and potential as ordinate whereby the heart disorder is detected from the waveform obtained relative to each point. However, according to an electrocardiograph of this system, it is difficult to accurately examine the whole electrical activity of the heart and according to a recently proposed electrocardiograph, 80 to 200 electrodes are applied to various points of the body surface near the heart and the potential of each point is measured, whereby the electrical activity of the heart is synthetically judged.

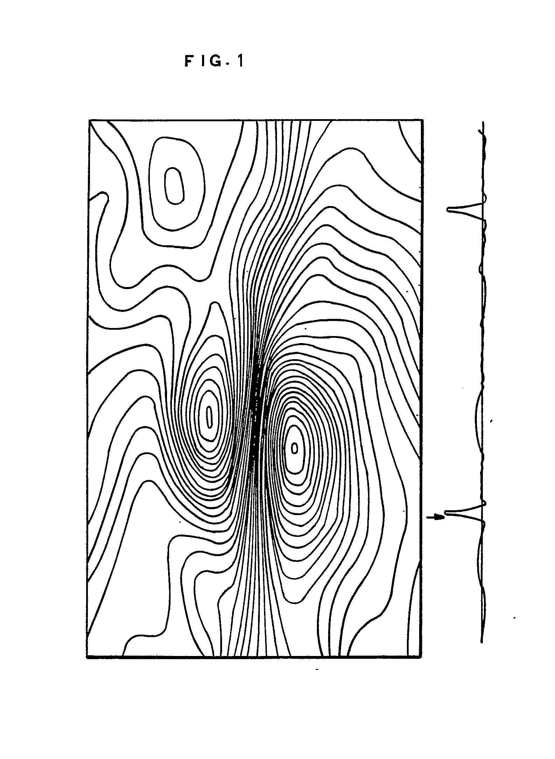

By this electrocardiograph, a potential map at a certain time is made relative to the body surface near the heart, as shown in Fig. 1. In this body surface potential map, the body surface potentials are shown as isopotential map so as to examine the distribution of the body surface potentials. This isopotential map is obtained e.g. by temporarily storing the potential of each electrode in a memory unit, calculating the isopotential point based on the potential of each electrode through a computer, and then drawing the isopotential line e.g. at a pitch of a few tens of microvolts on a television or an XY-plotter.

According to this electrocardiograph, a plurality of potential maps are obtained at intervals of the sampled time and the enlargement or contraction of the positively and negatively charged parts of the body surface near the heart are apparently recognized from the change of the potential gradient, whereby the electrical activity of the heart can be apparently indicated.

However, in the electrocardiograph of this kind, it is difficult to apply a number of electrodes to the points of the body surface with little contact resistance, and to stably and correctly detect the potential of each point.

For example, it takes four persons so long a time as 30 minutes to 1 hour to correctly place the conventional suction type electrodes to about 100 points of the body surface, and only one or two patients are examined in an hour even in the best case.

It is necessary that the electrodes of an electrocardiograph are applicable to any person, adult or child, male or female, having different body shape. The electrodes must be free of errors in potential measurement due to the unevenness of the body surface or its upward and downward motion caused by the breathing. Further, the electrodes are required not to give any terror, pains or sense of oppression to patients. Further, they are required to be applied or removed easily and rapidly, and to be easily maintained. Furthermore, it is necessary to place the electrodes correctly relative to one another or without deviation.

In detecting the potentials of the parts of the body surface, preferably all the electrodes are pushed onto the body surface with a strong force so as to stably and correctly measure the potentials of the electrode contact points. However, in an electrocardiograph for detecting the potentials of many points the human body, a number of electrodes are employed. And if the pressure applied by an electrode is 500 g and 100 electrodes are used, a force as strong as 50 kg in amount is applied on the chest. It necessarily gives a strong sense of oppression to patients. Therefore such an electrocardiograph is extremely unsuitable for the use of examining the heart of patients of declined strength.

Consequently, practically usable electrodes cannot be obtained only by increasing the pushing force of the electrodes.

According to a conventional electrocardiograph, since signals fed from each electrode are amplified and shown in a graph, the bad contact of electrodes can be easily judged in the output graph of the electrocardiograph.

However, with the proposed electrocardiograph, an isopotential map of the body surface at a sampled time is shown, and therefore, it is more difficult than in the conventional electrocardiograph to judge the heart disorder from bad contact of electrodes in the map. Consequently, in order to examine the heart with a high accuracy, all the electrodes must be always in sure electrical contact with the body surface. If a conventional suction type electrode or an adhesive tape type electrode comes into electrical bad contact for a minute per 100 minutes, either one of 100 electrodes is always in bad contact and accurate measurement cannot be performed.

It is thought that an electrocardiograph which shows an isopotential map of the body surface near the heart can indicate more accurately and apparently the electrical activity of the heart than a conventional electrocardiograph which shows only the change of the voltage of the measuring point. However, according to this electrocardiograph, potentials of a large number of points of the body surface must be detected simultaneously and surely. Since this problem was not sufficiently solved, an electrocardiograph of this system has not spread yet.

FR—A—2 088 780 discloses an electrode assembly for an electrocardiograph having a plurality of needle electrode assemblies for measuring potentials of a number of space points of a surface of a body. Each needle electrode assembly has a plurality of needle electrodes and is mounted on a rod which extends within a case and is urged outwardly by a coil spring so that the needle electrodes are urged in common by the coil spring.

An object of the present invention is to provide an electrocardiograph in which potentials of a number of spaced points of the body surface can be stably, surely and accurately detected and the electrical activity of patients of different body shapes can be measured in a short time.

According to the present invention there is provided an apparatus for measuring potentials of a number of spaced points on the surface of a body comprising a plurality of electrode assemblies each having a plurality of parallel needle electrodes resiliently urged in the same direction, an electronic circuit to which the electrodes are connected and for information processing of the potentials of the electrodes, and a monitor connected to the output of the electronic circuit for displaying a potential distribution map of the body surface, characterised in that the apparatus is an electrocardiograph, in that each electrode assembly comprises an electrode box out of which each needle electrode of that electrode assembly is urged by an individual resilient member in the direction of its length into contact with the surface of the body, in that the needle electrodes of each electrode assembly are grouped into electrode sets, in that the needle electrodes of each electrode set are electrically connected in parallel, and in that the distance between each needle electrode of an electrode set and the adjacent needle electrode of that electrode set is smaller than the distance between each electrode set and the adjacent electrode set.

The present invention provides an electrocardiograph in which if one needle electrode fails to be in sure contact with the body surface, an accurate potential distribution map can still be obtained.

The present invention provides an electrocardiograph in which the pushing force of a needle electrode applied to the body surface can be decreased and a potential distribution map of the body surface can be obtained without giving heavy pains and umcomfortable sense to patients.

An embodiment of the invention will now be described with reference to the appended drawings. These drawings are only for explanation and do not limit the scope of the present invention.

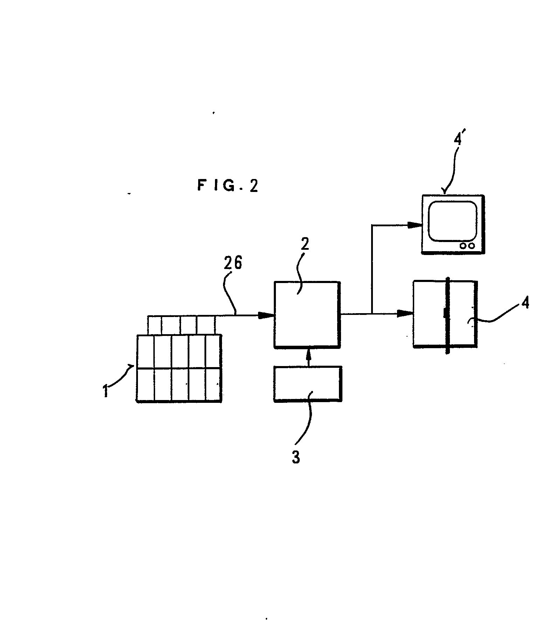

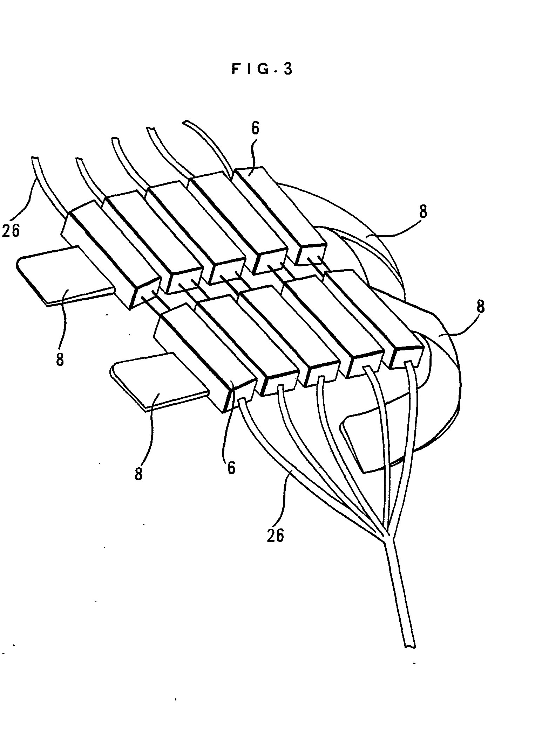

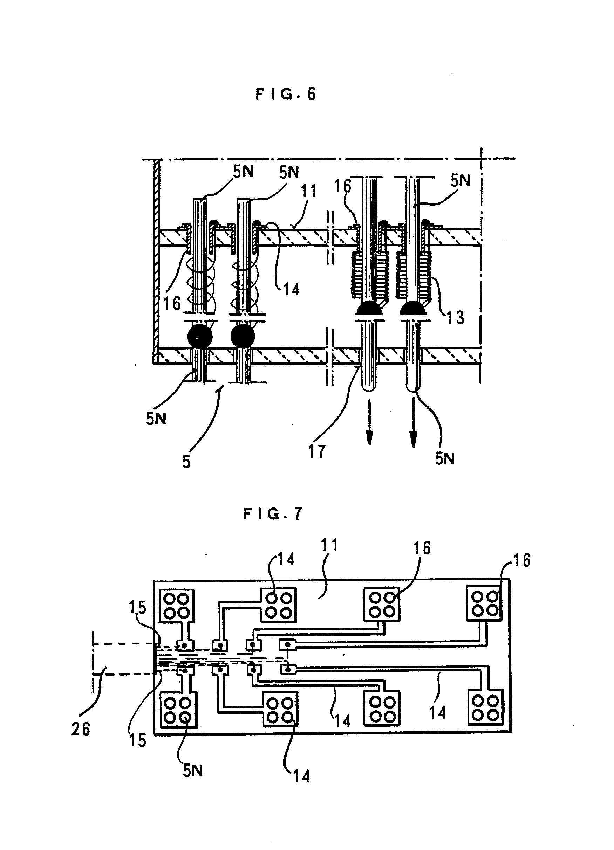

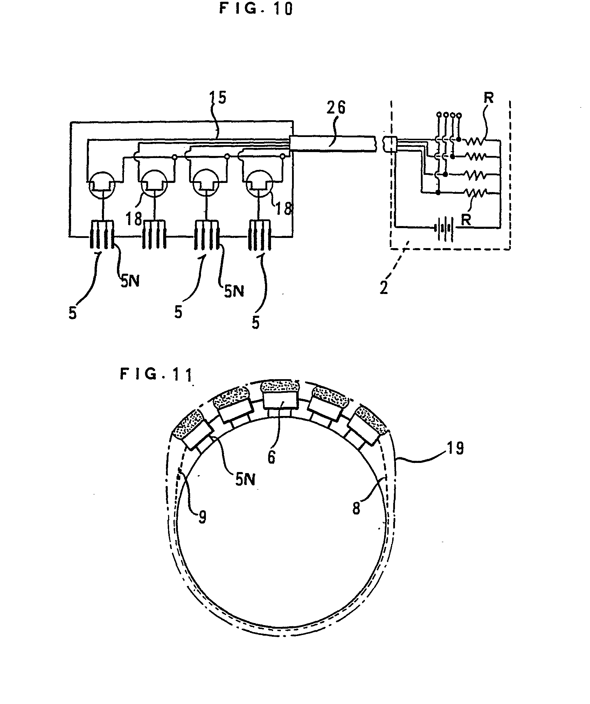

Fig. 1 is potential curves of the body surface near the heart; Fig. 2 is a block diagram of an example of electrocardiograph according to the present invention; Figs. 3 to 5 are perspective, sectional and bottom views of an example of electrode; Figs. 6, 8 and 9 are sectional views illustrate the arrangement of the stick electrodes; Fig. 7 is a plan view of a plate member; Fig. 10 is a connection diagram of a FET which is connected to the electrode; and Fig. 11 is a sectional view illustration the application of the electrode to the body surface.

An electrocardiograph in Fig. 2 comprises an electrode 1, an electronic circuit 2, an operational switch 3, an XY-plotter 4 and a monitor scope 4'.

As shown in Figs. 3 to 5, the electrode 1 comprises ten electrode boxes 6 and eight sets of stick electrodes 5 mounted on each electrode box.

The electrical boxes 6 are mutually connected through a movable member 7 in the form of a string-like rubber-like elastic member. To the outermost electrode box 6, an elastic winding band 8 is connected, and a connectable tape 9 is sewed onto the leading edge of the winding band 8.

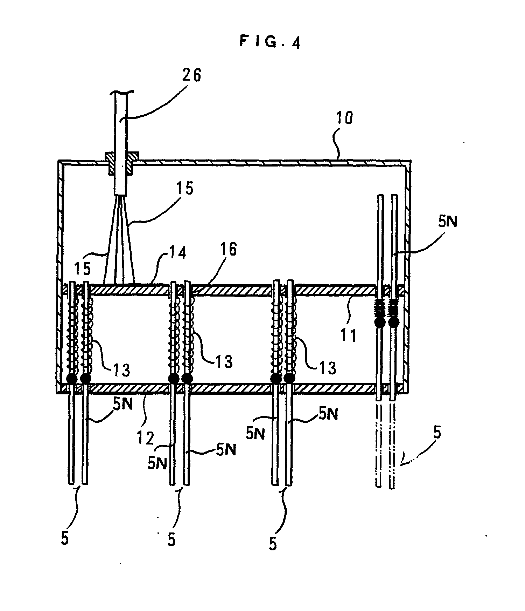

As shown in Fig. 4, the electrode box 6 is provided with the stick electrodes which are arranged to be movable in the axial direction.

The electrode box 6 comprises a downwardly opened box-like case 10 and two electrically isolated plate members 11, 12. Needle electrode 5N, being stick electrodes 5, are penetrated through the plate member 11, 12 so as to be movable inward and outward therethrough. Coil springs 13 are provided between the plate member 11, 12 so that each of the needle electrodes 5N is passed through the coil spring 13.

The coil spring 13 is a counter spring and its lower end is connected to the middle portion of the needle electrode 5N which its upper end is penetrated through the plate member 11 and connected to a conductor printed on the upper surface of the plate member 11.

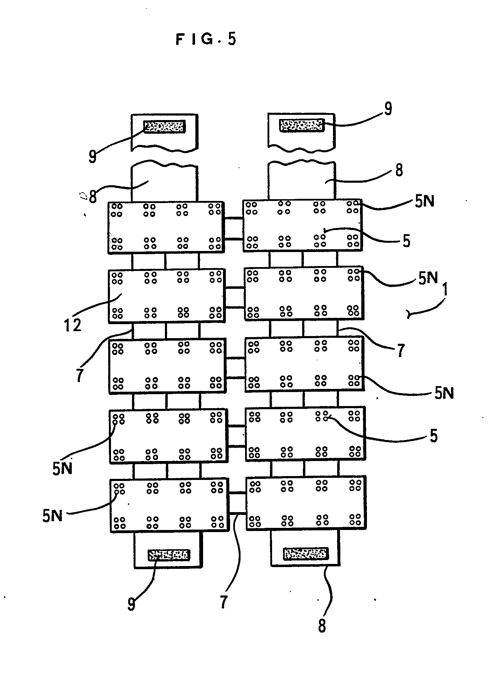

Each of the electrode boxes 6 shown in Figs. 5, 7 is provided with eight stick electrode sets 5, and a stick electrode set comprises four needle electrodes 5N.

Four needle electrodes 5N are arranged in close to one another. The space between the needle electrodes 5N is much smaller than that between the stick electrode sets. For example, the former is several to several tens of fractions of the latter. Four coil springs 13 through which the needle electrodes 5N are respectively passed are connected mutually by means of the conductor 14 provided on the upper surface of the plate member 11. According to this structure, even if either one of the needle electrodes 5N comes out of contact with the body surface, the potential of the body surface can be detected through another needle electrode 5N which is in contact with the body surface. Consequently, by an electrocardiograph having such a structure, the detection of the potential of the body surface can be especially ensured.

As shown in Figs. 5 and 7, if an electrode set comprises four needle electrodes 5N and a needle electrode comes into bad contact for 1 second per 100 seconds, the probability of all of the four needle electrodes coming in bad contact is 1 second per $10^8$ seconds i.e. close to substantially zero.

According to this structure, the space between the needle electrodes 5N is as small as 1 mm to 15 mm. Therefore, such a structure is an ideal one that the detected potential of the needle electrode 5N is transmitted to the lead wire 15 through the coil spring 13 for pushing out the needle electrode 5N, because each needle electrode is movable in the axial direction independently of the movement of other needle electrodes.

In the upper plate member 11, a tube member 16 is inserted into the through hole through which the needle electrode 5N is passed.

The tube member 16 comprises a metal tube or a tube with an inside surface being smooth and of low friction resistance, in order to lower the friction resistance between the tube member 16 and the needle electrode 5N which is a metal wire of stainless, copper, aluminium or conductive alloy. As shown in Fig. 6, the tube member 16 is somewhat protruded downwardly below the lower surface of the plate member 11. The upper end of the coil spring 13 is put on the protruded portion of the tube member 16 with the needle electrode 5N being pushed upwardly through the hole and the coil spring being pressed. According to this structure, when the needle electrode 5N is pushed to the uppermost position, the pressed coil spring 13 can be prevented from coming into contact with the needle electrode 5N and thus from restricting the movement of the needle electrode 5N. Therefore, the needle electrode 5N always can smoothly go in and out through the hole. The needle electrode 5N pushed out by means of the coil spring 13 is prevented from being slipped out in such a manner that the enlarged portion of the needle electrode 5N, to which the lower end of the coil spring 13 is fixed by solder joint or welding, is caught by the through hole 17 in the lower plate member 12. As shown in Fig. 7, the conductor 14 of copper layer or the like is printed on the upper surface of the upper plate member 11 and the lead wire 15 is connected to one end of the conductor 14.

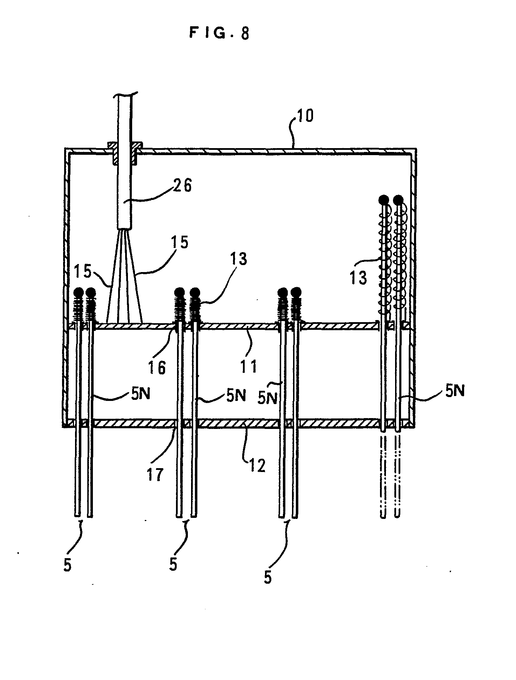

In the electrode box shown in Fig. 8, the coil spring 13 is provided on the upper end portion of the needle electrode 5N and above the upper plate member 11. And the needle electrode 5N is passed through the coil spring 13. The coil spring 13 is a tension spring. The upper end of the coil spring 13 is connected to the upper end of the needle electrode 5N with the lower end connected to the conductor printed on the surface of the plate member 11. And the lead wire 15 is connected to the conductor.

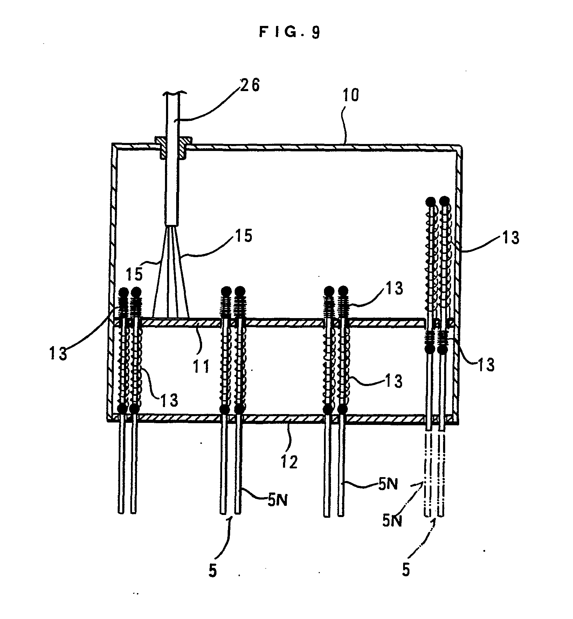

In the electrode box shown in Fig. 9, coil springs 13 are provided above and below the upper plate member 11. According to this structure, one end of either one or both of the upper and lower coil springs is connected to the conductor on the plate member 11, and the lead wire 15 is connected to the conductor. In this case, either one of the coil springs may be soft, that is, of a relatively low damping factor which indicates the power required for stretching the coil spring by a unit length.

The movable member 7 connecting the electrode boxes with one another may be formed of nonelastic string or belt, or soft and elastic one.

The lead wires 15 connected to the electrodes are gathered into an electrically shielded wire 26 and connected to the electronic circuit 2.

Since the potentials detected through the electrodes are considerably low, attention must be paid to removing of the external noises.

For this purpose, each block of electrode is separately shielded whereby the ratio of S to N can be improved. In order to further lower the noise level. it is favourable that a device amplifying the detected signal through the electrode, e.g. FET is provided within the block of electrode.

The connection of FET 18 and the stick electrode can be performed by providing a load resistance R of FET 18 within the electronic circuit as shown in Fig. 10, and thus it is unnecessary to provide a power supply within each electrode box. Further, it is convenient that the lead wires are not increased in number by the provision of FET. In other words, the electrode box, having eight stick electrodes, is provided with eight FET's and afford to transmit the detected body surface potentials through eight output signal lead wires and a grounded wire to the electronic circuit.

In Fig. 11, the electrodes are applied to the human chest surface. Each electrode box 6 is put on the body surface near the heart, and then the two ends of the winding band 8 is connected mutually by a connectable tape 9 whereby the electrodes of the electrode box 6 are applied to the body surface under a given pressure. In this step, the outside of the electrode box 6 may be further tightened by an elastic band 19 so as to push the electrodes more intensely onto the body surface.

The electronic circuit 2 is adapted to perform information processing of the electrical signal transmitted through the electrode according to a determined system, for example, to calculate the isopotential curves based on the electrical signal transmitted through the electrode at intervals of a sampled time, and then transmit the output signal thereof to the XY-plotter 4 and the monitor scope 4′ to show the isopotential maps of the body surface.

An electrocardiograph according to the present invention is available as an apparatus for detecting heart desease or detecting heart desease accompanied by other deseases in its early stages. And since, according to this electrocardiograph, the time required for examining a person is very short, the operation being very easy, parts thrown away after a measurement like an adhesive tape type electrode being few and thus the cost of the measurement being low, it is especially suitable for the group examination of heart desease.

**Claims**

1. Apparatus for measuring potentials of a number of spaced points on the surface of a body comprising a plurality of electrode assemblies each having a plurality of parallel needle electrodes (5) resiliently urged in the same direction, an electronic circuit (2) to which the electrodes (5) are connected and for information processing of the potentials of the electrodes (5), and a monitor (4) connected to the output of the electronic circuit (2) for displaying a potential distribution map of the body surface, characterised in that the apparatus is an electrocardiograph, in that each electrode assembly comprises an electrode box (6) out of which each needle electrode (5) of that electrode assembly is urged by an individual resilient member (13) in the direction of its length into contact with the surface of the body, in that the needle electrodes (5) of each electrode assembly are grouped into electrode sets, in that the needle electrodes (5) of each electrode set are electrically connected in parallel, and in that the distance between each needle electrode (5) of an electrode set and the adjacent needle electrode (5) of that electrode set is smaller than the distance between each electrode set and the adjacent electrode set.

2. An electrocardiograph according to claim 1 characterised in that each electrode set comprises two to six needle electrodes (5).

3. An electrocardiograph according to claim 1 or claim 2 characterised in that each needle electrode (5) is a metal pin.

4. An electrocardiograph according to any preceding claim is characterised in that it has 80 to 200 electrode sets in total.

5. An electrocardiograph according to any preceding claim in which each resilient member is a coil spring (13).

6. An electrocardiograph according to claim 5 characterised in that the coil spring (13) for each needle electrode (5) surrounds that needle electrode (5) and has one end electrically connected to the needle electrode (5), and in that each electrode set comprises a common conductor to which the other end of each coil spring (13) is connected.

7. An electrocardiograph according to any preceding claim characterised in that each electrode set comprises a preamplifier within the electrode box (6) and to which the needle electrodes (5) of that set are connected.

## Patentansprüche

1. Gerät zur Messung von Potentialen einer Anzahl von beabstandeten Punkten auf der Oberfläche eines Körpers, mit mehreren Elektroden-einheiten, die jeweils mehrere parallele Nadelelektroden (5) aufweisen, welche elastisch in derselben Richtung gedrückt werden, mit einer Elektronikschaltung (2), an welche die Elektroden (5) angeschlossen sind und welche eine Informationsverarbeitung bezüglich der Potentiale der Elektroden (5) durchführt, und einem Monitor (4) der an einen Ausgang der Elektronikschaltung (2) angeschlossen ist, um eine Potentialverteilungskarte der Körperoberfläche anzuzeigen, dadurch gekennzeichnet, daß das Gerät ein Elektrokardiograph ist, daß jede Elektrodeneinheit einen Elektrodenkasten (6) umfaßt, aus welchem heraus jede Nadelelektrode (5) dieser Elektrodeneinheit durch ein individuelles elastisches Element (13) in Richtung seiner Länge und in Berührung mit der Körperoberfläche belastet wird, daß die Nadelelektroden (5) jeder Elektrodeneinheit zu Elektrodensätzen gruppiert sind, daß die Nadelelektroden (5) jedes Elektrodensatzes elektrisch parallel geschaltet sind und daß der Abstand zwischen jeder Nadelelektrode (5) eines Elektrodensatzes und der benachbarten Nadelelektrode (5) dieses Elektrodensatzes kleiner ist als der Abstand zwischen jedem Elektrodensatz und dem benachbarten Elektrodensatz.

2. Elektrokardiograph nach Anspruch 1, dadurch gekennzeichnet, daß jeder Elektrodensatz zwei bis sechs Nadelelektroden (5) umfaßt.

3. Elektrokardiograph nach Anspruch 1 oder 2, dadurch gekennzeichnet, daß jede Nadelelektrode (5) ein Metallstift ist.

4. Elektrokardiograph nach einem der vorstehenden Ansprüche, dadurch gekennzeichnet, daß er 80 bis 200 Elektrodensätze insgesamt aufweist.

5. Elektrokardiograph nach einem der vorstehenden Ansprüche, dadurch gekennzeichnet, daß jedes elastische Element eine Schraubenfeder (13) ist.

6. Elektrokardiograph nach Anspruch 5, dadurch gekennzeichnet, daß die Schraubenfeder (13) für jede Nadelelektrode (5) diese Nadelelektrode (5) umgibt und an einem Ende elektrisch mit der Nadelelektrode (5) verbunden ist, und daß jeder Elektrodensatz einen gemeinsamen Leiter aufweist, an den das andere Ende jeder Schraubenfeder (13) angeschlossen ist.

7. Elektrokardiograph nach einem der vorstehenden Ansprüche, dadurch gekennzeichnet, daß jeder Elektrodensatz einen Vorverstärker innerhalb des Elektrodenkastens (6) umfaßt, an den die Nadelelektroden (5) dieses Satzes angeschlossen sind.

## Revendications

1. Appareil pour la mesure du potentiel d'un nombre de points espacés à la surface d'un corps, comprenant une pluralité d'ensembles d'électrodes ayant chacun une pluralité de tigés-électrodes (5) parallèles poussées élastiquement dans la même direction, un circuit électronique (2) auquel les électrodes sont reliées, et pour le traitement informatique des potentiels des électrodes 5, et un écran de surveillance (4) relié à la sortie du circuit électronique (2) pour présenter une carte de répartition du potentiel à la surface du corps, caractérisé en ce que l'appareil est un électrocardiographe, chaque ensemble d'électrodes comprend une boîte (6) d'électrodes hors de laquelle chaque tige-électrode (5) de cet ensemble d'électrodes est poussée dans le sens de sa longueur par un organe élastique individuel (13) jusqu'à son contact avec la surface du corps, les tiges-électrodes (5) de chaque ensemble d'électrodes sont groupées en jeux d'électrodes, les tiges-électrodes (5) de chaque jeu d'électrodes sont reliées électriquement en parallèle et la distance entre chaque tige-électrode (5) d'un jeu d'électrodes et la tige-électrode (5) voisine de ce jeu d'électrodes est plus faible que la distance entre chaque jeu d'électrodes et le jeu d'électrodes voisin.

2. Electro-cardiographe selon la revendication 1, caractérisé en ce que chaque jeu d'électrodes comprend 2 à 6 tiges-électrodes (5).

3. Electro-cardiographe selon la revendication 1 ou 2, caractérisé en ce que chaque tige-électrode (5) est une tige métallique.

4. Electro-cardiographe selon l'une quelconque des revendications précédentes caractérisé en ce qu'il comprend 80 à 200 jeux d'électrodes au total.

5. Electro-cardiographe selon l'une quelconque des revendications précédentes dans lequel chaque organe élastique est un ressort de compression (13).

6. Electro-cardiographe selon la revendication 5, caractérisé en ce que le ressort de compression (13) de chaque tige-électrode (5) entoure cette tige-électrode (5) et a une extremité reliée électriquement à cette tige-électrode (5), et chaque jeu d'électrodes comprend un conducteur commun auquel l'autre extrémité de chaque ressort de compression (13) est reliée.

7. Electro-cardiographe selon l'une quelconque des revendications précédentes caractérisé en ce que chaque jeu d'électrodes comprend à l'intérieure de la boîte d'électrodes (6) un pré-amplificateur auquel les tiges-électrodes (5) de ce jeu sont reliées.

FIG.1

FIG. 2

FIG.3

**F I G . 4**

FIG. 5

FIG.6

FIG.7

FIG.8

FIG.9

FIG.10

FIG.11